# EUROPEAN PATENT SPECIFICATION

(11) **EP 2 975 961 B1**
(45) Date of publication and mention of the grant of the patent: **09.08.2017**
(21) Application number: 14715710.1
(22) Date of filing: 21.03.2014
(51) Int. Cl.: A41D 19/015, A63B 71/14, A61F 5/058

(54) **FINGER PROTECTION DEVICE**
FINGERSCHUTZVORRICHTUNG
DISPOSITIF DE PROTECTION DE DOIGT

(30) Priority: 21.03.2013 NL 2010497
(43) Date of publication of application: 27.01.2016
(73) Proprietor: InnoSafety B.V., 2566WR Den Haag (NL)
(72) Inventor: Smit, Pieter Casper, 2566 VH Den Haag (NL)
(74) Representative: Nederlandsch Octrooibureau
(86) International application number: PCT/NL2014/050175
(87) International publication number: WO 2014/148906

(56) References cited:
- DE-A1-102007 043 823
- FR-A1- 2 647 314
- US-A- 4 524 464
- US-A- 4 653 490

## Description

### BACKGROUND OF THE INVENTION

### 1. Field of the Invention

The invention relates to a finger protection device to prevent finger injuries during activities with large stresses and strains on the ligaments in the hand. Furthermore, the invention relates to a thumb protection device to prevent thumb injuries. In addition, the invention relates to a glove or mitten with such a finger protection device.

### 2. Description of the Related Art

Hand injuries in general tend to be disregarded by both patients and doctors - until it happens to oneself. Never was this more the case than in thumb injuries, as the importance of the thumb is usually underappreciated until it is injured. The thumb allows the movement of opposition - against the index finger - and this gives the ability to grip with the hand. Losing this function means losing basic skills that are taken for granted - like holding a pen, a knife/fork or spoon or undoing buttons on a shirt.

Injuries of the thumb and hand are fairly common in sporting activities as in daily (professional) life. In skiing injuries of the thumb are reported to be the second most injury after injuries affecting the knee. Thumb injuries are reported to be between 5 and 15 % of all injuries in Alpine skiing and are sustained by approximately 80,000 - 240,000 patients per year world wide.

The ski-thumb is an injury affecting the Ulnar Collateral Ligament (UCL) at the metacarpophalangeal joint of the thumb. The mechanism is due to forces after a skier fall with the pole in their hand. The handle of the pole will act as a fulcrum applying forces across the joint and thereby putting the ligament under strain. Consequently it may tear completely (grade 3), or partly (grade 2 or 1) or may cause a fracture of the ulnar base of the proximal phalanx of the thumb.

The thumb comprises of three bones - the distal phalanx, the proximal phalanx and the metacarpal bone. The latter forms a join with the trapezium bone in the hand as shown on the right. The thumb also has one very important joint when it comes to snow sports - the metacarpophalangeal (MCP) joint. This joint is supported and stabilized on the inside (lateral side) by the all important ulnar collateral ligament. It is the MCP joint - and the UCL in particular, that are involved and injured in the classic ski injury.

Various devices have been designed in order to reduce the incidence of and the risk on getting a ski-thumb in ski accidents. Adjustments to the ski pole and pole clipping, like pole clipping directly into the glove, or strapless poles were developed. The theory that losing the pole before the skier will hit the ground without the pole in the hand, and therefore the mechanism of the pole forcing the thumb outwards is excluded will theoretically reduce the risk on UCL injuries.

Nevertheless the skier will hit the surface with high velocity, resulting in high energy trauma. If the hand is stretched out, which is a natural reflex, the skier still has a significant change that the thumb will be forced outwards due to hitting the ground, resulting in a fracture or UCL injury. This might be an explanation that several studies showed no significant decrease in incidence of thumb injuries yet, even since the introduction of the ski pole adjustments.

In addition, the American publication US8066655 describes a protective glove comprising a brace element that encloses the user's hand. A spanning portion of the brace element spans across the dorsal side of the hand to encircle the metacarpal region of the fingers. The spanning portion includes a thumb opening and a grouped finger opening. The brace element encapsulates the proximal phalanx of the thumb and most of the thumb's metacarpal bone. A disadvantage of this protective device / glove is immobilisation of the proximal thumb by the brace element. This device makes it impossible to move the thumb in any direction. The distal phalanx however is free of protection and can be moved in any direction but is not protected and therefore prone to be injured. Forces (medial, lateral, axial, etc) can easily result in fracture, rupture, strain, luxation, bruises of (parts) of the thumb.

A disadvantage of this protective glove is the immobilisation of the thumb by the brace element. During activities such as sports, it is desired that the thumb can be used in full, but without the risk of an injury by overstretching or overstraining. Additionally, the ability to feel of the hand and thumb is important in handling and therefore the device should be designed to guarantee this.

Furthermore, forces exerted in the longitudinal direction of the thumb or other fingers still have to be intercepted by the fingers themselves, such that injuries to the ligaments and bones of the fingers, in particular of the UCL and the proximal phalanx of the thumb, will be incurred.

German patent document DE102007043823A1 describes a finger protection device with a thumb receiving part that is hingeably coupled to a mounting plate, which in turn is fixable to a rear region of a hand. Natural movements of the thumb and the fingers are allowed, but the mounting plate comprises a stop unit to delimit a pivoting range for the thumb receiving part.

It would be desirable to provide a finger protection device that alleviated at least some of the perceived inconveniences of the prior art.

### BRIEF SUMMARY OF THE INVENTION

According to the invention there is provided a finger protection device, comprising a hand brace element for accommodating at least a carpal region of a hand, and a finger brace structure comprising at least one finger top cap to accommodate a finger top and a flexible connecting element extending between the hand brace element and the finger top cap, the connecting element being sufficiently rigid to transmit an axial first force applied to the finger top cap to the hand brace element.

The hand brace element accommodates at least the carpal region extending over the carpals of the hand such that it provides support to the carpal region. Preferably, the hand brace element accommodates the metacarpal region extending over the metacarpals of the hand as well. In addition, the hand brace element will accommodate and support the ligaments joining these bones to form the palm of the hand. Furthermore, the hand brace element forms the base of the finger protection device to which the finger brace structure is connected.

The finger brace structure comprises a connecting element connected to the hand brace element, and a finger top cap. With finger all five digits, i.e. including the thumb, are meant. The finger top cap accommodates the top of one finger, i.e. the distal phalanx, and extends over at least part of the distal phalanx of the finger, preferably over the entire distal phalanx of the finger.

The connecting element forms a flexible connection between the hand brace element and the finger top cap. The flexibility of the connecting element should be such that when a user wishes to bend the finger provided with the finger brace structure, the connecting element can be moved without too much effort. Additionally the connecting element is sufficiently rigid to transmit an axial force applied to the finger top cap, i.e. parallel to the connecting element, to the hand brace element. Such a force can be exerted on the finger protection device when the user falls with stretched out hands and fingers to the ground, while landing on the top of the finger.

When during a fall the hand hits the ground, the ligaments in a thumb or any other finger can be overstretched. Since the UCL will rupture when the thumb is at an angle of an angle of at least 33° ± 9° (degrees) and at a force of 2*10³± 1*10³ N-mm, i.e. from about 1*10³ N-mm to about 3*10³ N-mm, the device should be able to withstand such a force and deflect the force to the hand. The hand will be forced to move in a radial (lateral) direction such that the force of the fall is deflected to the wrist and subsequently to the (lower) arm. The elbow and the shoulder will move upon receipt of the force by the arm.

The connecting element may have anisotropic, i.e. non-symmetric, properties. These properties can be achieved by using, for example but not limited to, a material with a relatively low bending resistance and a relatively high buckling resistance. Alternatively, a construction with the desired properties can be used, as described further below.

The connecting element preferably comprises a U-shaped profile of which a base covers the dorsal side of the phalanges and sides extending from the base cover part of the lateral and medial side of the phalanges of the finger, thereby leaving a part of the lateral and medial side of the phalanges and palmar side of the phalanges free or uncovered.

The connecting element preferably only covers part of the finger and can be designed in a 'U-profile' covering the dorsal side of the finger and part of the lateral and medial side of the finger, thereby leaving a part of the lateral and medial side of the finger and palmar side of the finger free. The U-profile provides a) the possibility of maintaining the feeling (tactile) of the skin and b) the possibility to move or bend the finger towards the palm of the hand, but c) keeping rigidity in the opposite direction, such to prevent overstretching of the finger ligaments.

Bones in a hand are more fragile compared to bones in an arm or a leg, mainly because of the small sizes of these bones. The skeleton of the human hand consists of 27 bones: the eight short bones of the carpus, the carpal bones or carpals, organized into a proximal row (scaphoid, lunate, triquetral and pisiform), which articulates with the skeleton of the forearm, and a distal row (trapezium, trapezoid, capitate and hamate), which articulates with the bases of the metacarpal bones (i.e. the bones of the palm or "hand proper"). Together with the fourteen phalanx bones or phalanges of the fingers these metacarpal bones form five rays or poly-articulated chains.

The articulations in the hand comprise the interphalangeal articulations of the hand (the hinge joints between the finger bones), the metacarpophalangeal joints (where the fingers, including the thumb, meet the palm) and the intercarpal articulations (where the palm meets the wrist).

The bones of the hand are mutually connected by ligaments that allow the bones to articulate with respect to each other, resulting in for example a gripping movement of the hand and fingers. Any hard force on the thumb, or any finger, that pulls the thumb away from the hand (called a valgus force) can cause damage to the ulnar collateral ligaments. When the thumb is straight, the collateral ligaments are tight and stabilize the joint against valgus force. If the force is too strong, the ligaments may tear. They may even tear completely. A complete tear is also called a rupture. When the collateral ligaments actually tear, the MCP joint becomes very unstable. It is especially unstable when the thumb is bent backwards towards the dorsal side of the hand. If one of the ligaments pulls away from the bone and folds backwards, it won't be able to heal in the correct position to the bone. When this happens, surgery is needed to fix the ligament to the bone again. Sometimes the ligament itself will not tear but instead pulls a small piece of bone off the base of the thumb where it is attached. This is called an avulsion fracture. If the fracture does not heal correctly, this could lead to an unstable thumb joint.
In cadaver studies the strength and stiffness of the UCL is reported to failure at 2,311.7± 1,152.8 N-mm (1,158.9 - 3,464.5 N-mm), with a rotational stiffness of 76.7 ± 57 N-mm ° (degree) and at an angle of 33° ± 9° (degrees).

According to an embodiment, the finger brace structure is sufficiently rigid to transmit a transverse second force on the connecting element directed to the dorsal side of the hand brace element, to the hand brace element. The finger brace structure can be relatively rigid in an axial direction of the connecting element and relatively flexible in a transverse direction to the connecting element and directed to a dorsal side of the phalanges of the finger. Preferably, the rigidity of the finger brace structure and/or the connecting element is such that any force exceeding the maximum strength and/or (rotational) stiffness of the UCL will be deflected towards the hand brace element by the finger brace structure, such that injury of the UCL is prevented.

Flexion, wherein the angle between the bones of the limb, i.e. the finger, and the palm at a joint decreases, of a finger can occur in the direction of the palm, i.e. the fingers are moved towards the palm, to form a fist. In the direction away from the palm, i.e. in the direction of the dorsal side of the hand, i.e. the transverse second force, flexion is limited by the ligaments between the metacarpals and the proximal phalanges. In case the finger is flexed to touch the dorsal side of the hand, the ligaments are most likely to be overstretched and can be torn. To prevent such an injury to the hand, the finger brace structure, and more particular the connecting element, can be sufficiently rigid to transmit a force that will flex the finger towards the dorsal side of the hand, to the hand brace element.

According to a further embodiment, the connecting element comprises a movable connection between the finger top cap and the hand brace element for allowing movement of the finger in a direction towards a palm of the hand in use. The movable connection can be the result of the use of a flexible material, for instance when the finger protection device comprises a single unit where the finger brace structure and the hand brace element are made of one piece. In such a case, the finger brace structure can be deformed when in use a transverse third force is exerted on the finger brace structure, e.g. by the finger to form a fist of the hand, or by an external source where the force is directed to a dorsal side of the finger brace structure. Alternatively, the finger brace structure and the hand brace element can be separate parts of the device that are connected by a pivotable connection, such as a hinge, such that the connecting element is pivotable with respect of the hand brace element when the third force is exerted on the finger brace structure, e.g. to form a fist or to make a gripping movement with the hand.

According to another embodiment, the connecting element comprises a connecting element part for each phalanx in a finger, wherein the connecting element part in use covering a distal phalanx of a finger is connected to the finger top cap, and wherein the connecting element parts are connected to each other by a pivotable connection, such that the connecting element parts are pivotable with respect to each other when in use the third force is exerted on the finger brace structure.

The connecting element can be built from separate connecting element parts that each cover an individual phalanx of the finger. For fingers other than the thumb the number of connecting element parts will be three, as the index, middle, ring and small finger all have three phalanges, for the thumb two, as the thumb has two phalanges.

The connecting element parts can be pivotably connected to each other and the hand brace element at a pivot point, for instance at each respective end of a respective leg of the U-profile described above.

The movable connection between the respective connecting element parts forming the connecting element, and/or the connection between the connecting element and the hand brace element can be established by at least one flexible elongated connection member connected with a first and a second end to a connecting element part. Preferably, at least three parallel flexible elongated connection members, such as wires, tubes, rods, having an appropriate diameter, or strips of material having a high width to length ratio, are provided. The higher the number of parallel elongated connection members, the more resistance against torsion or transverse forces of the connecting element at the pivotable connections is provided. Resistance against torsion increases the stiffness in transverse direction, in torsion of the connecting element and therefore adds to the protection of the finger. A preferred number of elongated connection members is five, more preferred is seven or even higher.

The flexible elongated connection members are preferably slidably received in through holes forming receiving openings in the respective connecting element parts. Upon movement of the finger, the connecting element parts can move with respect to each other by the elongated connection members sliding in the respective through holes. The length of the elongated connection members is such that upon normal movement of the respective finger, at least the ends of the elongated connection members will remain in the through holes to retain the movable connection. The end of the elongated connection members can be made such that the movement of the elongated connection members within the through holes will be blocked with a blocking member, for instance an end portion with a thickening or an end portion with a larger diameter or a bend or knot in the end portion of the elongated connection member. Blocking of the movement is to maximize flexion of the finger but preventing over flexion of the finger.

In case of one elongated connection member, the elongated connection member can be a single flexible sheet extending over at least a portion of the connecting element parts, such as a flexible and/or elastic lining, to establish the flexible connection in a similar way. The connecting element parts can be pivotably connected to each other and the hand brace element with the lining at the inside of each respective connection element part, i.e. the side facing the finger or palm of hand when in use. The lining can cover at least a first and a second end of adjacent connecting element parts, or can be a sheet covering the inside of the connecting element from the hand brace element to the connecting element part covering the distal phalanx, forming a lining extending along the inside of the connecting element. The lining then forms a flexible and/or elastic connection between each connecting element part and between the connecting element and the hand brace element. The lining can be a textile, a sheet or the like made of natural or synthetic materials, such as a polymer material. The advantage of the lining is that it can absorb or divert forces acting on the finger concerned, contributing to the strength of the device and preventing overstretching of the finger concerned. Alternatively, a combination of the lining and the wires, rods or tubes can be used.

The connecting element part covering the distal phalanx can be integrated within the finger top cap. The pivotable connection allows at least pivoting towards the palm of the hand. Preferably, the pivotable connection is prohibited to pivot towards the dorsal side of the hand, wherein the connecting element attains a rigid position upon exertion of the second force directed to the dorsal side of the hand.

A web space part is provided to cover at least part of a web space between the thumb and the index finger. Additionally, web space parts can be provided to cover each web space between a pair of fingers. The finger top cap extends from the top of the distal phalanx to the web space part to provide a closure of the connecting element at the palmar side of the finger, in particular of the thumb. The finger top cap, particularly the embodiment closing the connecting element, has the advantage that the scooping effect of the connecting element is reduced when a user falls down in the snow and adds to comfort, stabilization, i.e. safety and handling of the device. It is preferred that the web space part is made of a textile like material or leather to provide comfort to the user of the finger protection device. In case the finger top cap extends from the top of the distal phalanx to the web space part, it is preferred that the finger top cap and the web space part are made of the same material.

The pivotable connection between the connecting element parts and/or the hand brace element can be established by a connecting strip provided at the respective edges of the connecting element parts. The connecting strip can extend along the edges of the connecting element parts from the hand brace element to the finger top cap. It is preferred that the connecting strip is at least flexible at a location where the connecting element parts are movably connected to each other. The connecting strip is preferably formed by a piping and is preferably made of a textile material or leather. A similar piping can be provided at the edges of the web space part.

The connecting strip can also be provided as a piping at the respective edges of a connecting element made of a single piece, i.e. not made of several connecting element parts. The piping should then be sufficiently flexible such that the flexibility of the connecting element is not decreased. Additionally, the connecting strip can be used to deflect any forces of a (forced) abduction of the thumb with or without extension, or over-flexion of the other finger(s), to the hand brace element.

To prevent snow and ice entering the connecting element parts, in particular when the finger protection device is worn over a glove or mitten, a sealing element, such as a sheet of flexible material, can be provided between or covering the respective connecting element parts and/or hand brace element. The flexibility of the sealing element ensures the flexibility of the connecting element upon movement of the respective finger in the connecting element.

According to a further embodiment, the pivotable connection comprises a blocking element blocking pivoting of the connection element when in use the axial first force is exerted on the finger brace structure. The blocking element prevents the connecting element parts to pivot towards the dorsal side of the hand. Additionally, the blocking element can act to form a rigid connecting element from the connecting element parts, which connecting element can transmit the axial first force applied to the finger top cap to the hand brace element.
Preferably, the blocking element is formed at the opposite longitudinal ends of a respective connecting element part by a relatively thicker portion extending along the ends of the connecting element part. Such a relatively thicker portion can also be provided at the connecting element part forming an integral part with the hand brace element. The connecting element part covering the distal phalanx of the finger in use can be provided with such a thicker portion at the end facing an adjacent connecting element part. The other end of the connecting element part covering the distal phalanx is attached to the finger top cap. The relatively thicker portions are provided with abutment surfaces where the adjacent connecting element parts abut each other upon movement of the thumb away from the palm of the hand, such that overstretching is prevented. According to another embodiment, the connecting element covers at least one of a dorsal side and a palmar side of phalanges of the finger, preferably the connecting element covers just the dorsal side of the phalanges of the finger. The connecting element extends between the hand brace element and the finger top cap. In order to obtain sufficient flexibility by other means than material choice, the freedom of movement of the finger can be maximized by covering at least one of the palmar and dorsal sides of the phalanges of the finger. This means that when both the palmar side and the dorsal side of the phalanges are covered, the medial and lateral sides of the phalanges are uncovered by the connecting element, i.e. the connecting element does not cover the entire periphery of the phalanges. To increase the movement of the finger even more, the connecting element can cover only one of the palmar side or the dorsal side of the phalanges. Preferred is that the connecting element only covers the dorsal side of the phalanges. This embodiment facilitates bending of the finger to form a fist or to hold an object without having any material in between the fingers and the palm or the object and maintain the ability to feel by the finger.

Alternatively, the finger brace structure can comprise a covering element to cover the parts of the phalanges not covered by the connecting element to obtain additional stiffness in the finger brace structure.

According to the invention the hand brace element comprises an entry opening for inserting the hand and at least two exit openings for extension of the fingers of the hand in use. Preferably, the hand brace element comprises at least an exit opening for a thumb and an exit opening for grouped fingers are provided, wherein the finger brace structure is provided at the thumb opening for protection of the thumb. In case the hand brace element comprises an exit opening for the thumb and an exit opening for the other four grouped fingers (so one opening for four fingers), the finger brace structure can be provided at the exit opening of the thumb alone. In case all fingers require protection by the finger protection device, the hand brace element can be provided with exit openings for each individual finger and a finger brace structure can be provided at each exit opening of a finger, including the thumb. Other combinations of exit openings and finger brace structures are possible as well. Each finger brace structure is meant for one individual finger and is combined with an exit opening for that individual finger.

According to a further embodiment, the hand brace element is sufficiently rigid to receive at least one of the axial first force and the transverse third force from the finger brace structure. As the hand brace element acts as a base for the finger protection device, it is preferred to be able to receive the transmitted forces from the finger brace structure. As described above, the hand brace element supports at least the carpal region and preferably also the metacarpal region of the hand. Therefore, it is preferred that the hand brace element is sufficiently rigid to support at least the carpal region of the hand such that during falling the injury to at least the carpal region and preferably also the metacarpal region is decreased or prevented.

According to an embodiment, the hand brace element comprises an adjusting element at a dorsal side for adjusting a width dimension of the hand brace element. The hand brace element can be used by different users. The size of the hands of the users can be different. In order that the finger protection device can be fitted to the hands of each user, the hand brace element can be adjusted to the width of each hand to provide sufficient support to the finger brace structure.

In addition, the invention relates to a thumb protection device, comprising a hand brace element for accommodating at least carpal region of a hand, and a thumb brace structure comprising a thumb top cap to accommodate a thumb top and a flexible connecting element extending between the hand brace element and the thumb top cap, the connecting element being sufficiently rigid to transmit an axial force applied to the thumb top cap to the hand brace element.

In a preferred embodiment of the thumb protection device, the connecting element comprises a connecting element part for each phalanx, wherein the connecting element part in use covering a distal phalanx is connected to the thumb top cap, each connecting element part comprising a U-shaped profile covering the dorsal side of each phalanx and at least part of a lateral and at least part of a medial side of each phalanx, wherein the connecting element parts are connected to each other by a pivotable connection through a connecting strip provided at respective ends of respective legs of the U-shaped profile, such that the connecting element parts are pivotable with respect to each other when in use the third force is exerted on the thumb brace structure, at least one pivotable connection comprising a blocking element blocking pivoting of the pivotable connection beyond a flexed position of the hand in response to a force on the thumb towards the dorsal side of the hand, wherein each blocking element is formed by relative thicker portion at respective ends of each connecting element part, each portion provided with abutment surfaces where the adjacent connecting element parts abut each other in use upon movement of the thumb away from the palm of the hand, the thumb protection device further comprising a web space part covering at least part of a web space between the thumb and an index finger, wherein the thumb top cap extends from the top of the connecting element part covering the distal phalanx of the thumb to the web space part to provide a closure of the connecting element at the palmar side of the thumb.

The thumb protection device protects the thumb in particular against injuries as described above. The thumb is a very important finger or digit, as the thumb allows one to hold an object to perform an action. Without the use of a thumb many daily and obvious acts could not be performed. A preferred embodiment of the finger protection device as described above is therefore the thumb protection device.

Furthermore, the invention relates to a glove or mitten comprising a finger protection device as described above or a thumb protection device as described above. The finger protection device or the thumb protection device can be inserted into an existing glove or mitten, i.e. the glove or mitten and the device are two individual parts that can be assembled later, e.g. after manufacturing. Preferably, the device and the glove or mitten form an integral unit. For instance, the glove or mitten can be manufactured around or within the pre-made device.

### BRIEF DESCRIPTION OF THE DRAWINGS

The features and advantages of the invention will be appreciated upon reference to the following drawings of a number of exemplary embodiments, in which:
Figure 1 shows a view to the palmar side of a hand of a first embodiment of the device according to the present invention;
Figure 2 shows a view to the dorsal side of a hand of a first embodiment of the device according to the present invention;
Figure 3 shows a perspective view of a first embodiment of the device according to the present invention;
Figure 3a shows a detail of the device of Fig. 3.
Figure 4 shows a view to the dorsal side of the hand of a second embodiment of the device according to the present invention;
Figure 5 shows a view to the dorsal side of the glove according to the invention.
Figure 6 shows a side view of a third embodiment of the device according to the present invention.
Figure 7 shows a view of an inside of the third embodiment of Fig. 6.
Figure 8a and b show a cross-sectional view of the third embodiment of Fig. 6 and 7.

### DESCRIPTION OF ILLUSTRATIVE EMBODIMENTS

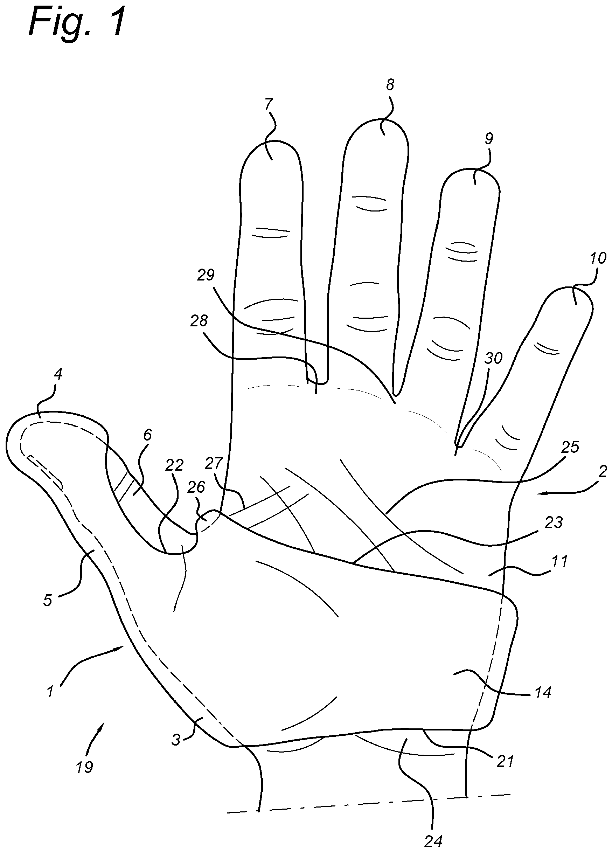

Figure 1 shows a view to the palmar side 11 of a hand 2 with a finger protection device 1 for the thumb 6, i.e. a thumb protection device. The thumb protection device 1 comprises a hand brace element 3 supporting the carpal region 24 and the metacarpal region 25 of the hand palm 11. The hand brace element 3 comprises an entry opening 21 for inserting the hand 2 into the hand brace element 3, an exit opening 23 for grouped fingers 7-10, and an exit opening 22 for the thumb 6. The palmar side 14 of the hand brace element covers the carpal region 24 and the metacarpal region 25 of the hand palm 11.

In between the exit openings 22, 23 a web space part 26 is provided. The web space part and the hand brace element can be made as one single piece. Additionally, the web space can be made of the same material or can be made of another material and is preferred to be flexible and/or adjustable. This web space part 26 covers at least part of the web space 27 between the thumb 6 and the index finger 7. Between each two fingers 7-10 the hand comprises a web space 28-30, respectively. The web space part 26 adds to the rigidity of the hand brace element 3. Furthermore it stabilizes the connecting element 5 and thumb top cap 4 and therefore the thumb 6. The web space part 26 can be made out of one piece and of the same material as the finger protection device 1. Alternatively, the web space 26 can be made of another material and made as a single piece with the hand brace element or assembled from separate pieces.

The finger protection device further comprises a finger brace structure 19 comprising a connecting element 5 and a finger top cap 4. The connecting element 5 connects the finger top cap 4 with the hand brace element 3 and covers the dorsal side and part of the medial and lateral sides of the thumb phalanges. The finger top cap 4 covers part of the distal phalanx of the thumb 6.

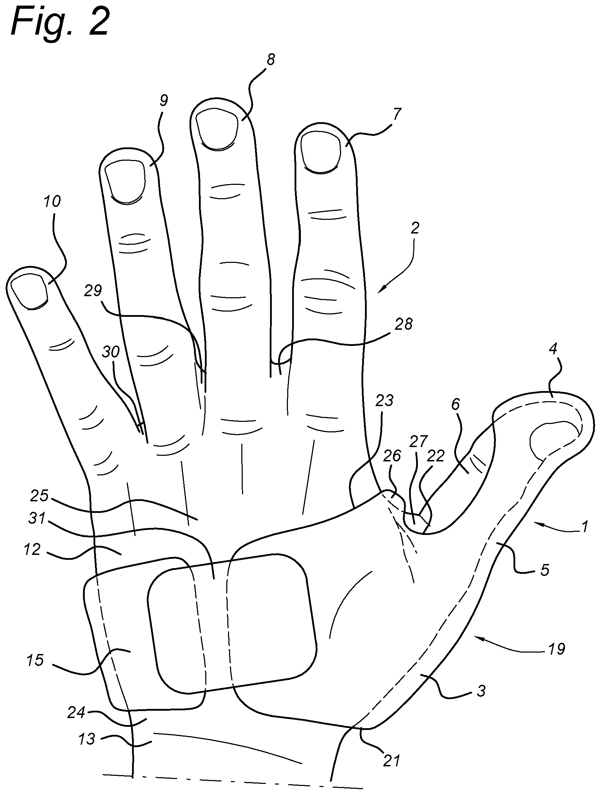

Figure 2 shows a view to the dorsal side 12 of a hand 2 with a finger protection device 1 for the thumb 6. The dorsal side 15 of the hand brace element 3 has an adjustment opening 31 such that the width dimension of the hand brace element can be adjusted for wider or narrower hands. The adjustment opening 31 can be provided with an adjustment element (not shown) to fix the adjusted width desired by the user. The hand brace element 3 covers the dorsal side 12 of the hand 2 to a distance from the wrist 13. This allows movement of the wrist 13 to move the hand 2 in its entirety with respect to the arm.

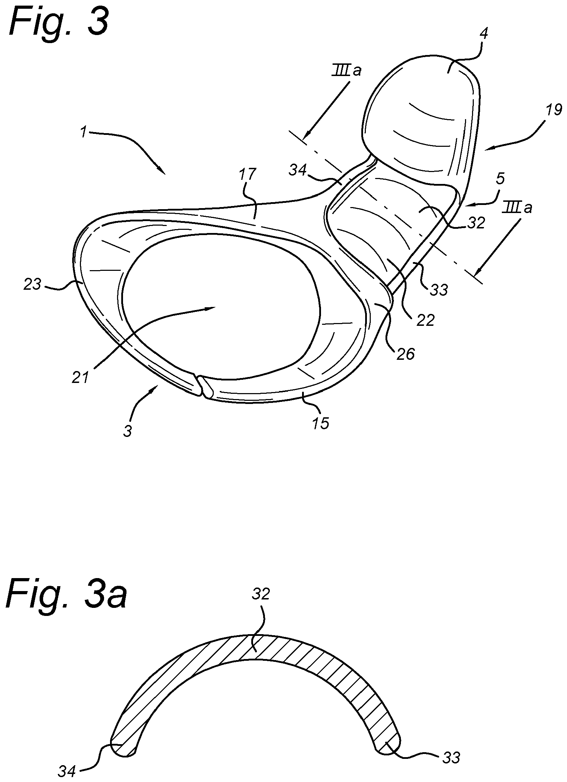

Figure 3 shows a perspective view of the finger protection device 1 without a hand 2 inserted. The hand brace element 3 comprises an enclosing element that surrounds the hand palm 11 and dorsum 12 and covers the carpal region 24 and metacarpal region 25 of the hand 2. The finger brace structure 19 extends from the hand brace element 3 and comprises the finger top cap 4 and the connecting element 5 connecting the hand brace element 3 and the finger top cap 4. The connecting element 5 comprises a U-shaped profile, as shown in the cross sectional detail of figure 3, covering the dorsal side of the phalanges and part of the medial and lateral sides of the phalanges of the thumb. The U-shape comprises a curved base 32 and sides 33, 34 extending from the curved base 32. The U-shape of the profile provides rigidity of the connecting element, and therefore for the finger brace structure, for movement of the thumb directed to the dorsum of the hand, i.e. for overstretching, as the sides 33, 34 of the U-shape covering the medial and lateral sides of the thumb and the curved base 32 will prevent the U-shape to bend towards the dorsum of the hand. In addition, the U-shape of the connecting element provides for rigidity in an axial direction of the connecting element, i.e. in a direction along the phalanges. Furthermore, the U-shape provides flexibility for the connecting element for movement of the thumb directed to the palm of the hand, e.g. for a gripping movement or to form a fist.

The hand brace element 3 is provided with an entry opening 21 for inserting a hand, an exit opening 23 for the group of the index finger 7, middle finger 8, ring finger 9 and pinky 10, and an exit opening 22 for the thumb 6. The two exit openings 22, 23 are separated by a web space part 26 that covers the web space 27 between the thumb 6 and the index finger 7. Other web spaces 28-30 are between the index finger 7 and the middle finger 8, the middle finger 8 and the ring finger 9, and the ring finger 9 and the pinky 10, respectively. The web space part 26 provides additional rigidity of the hand brace element 3, in particular when a force directs the thumb 6 to the dorsal side 12 of the hand 2, i.e. in case of overstretching of the UCL.

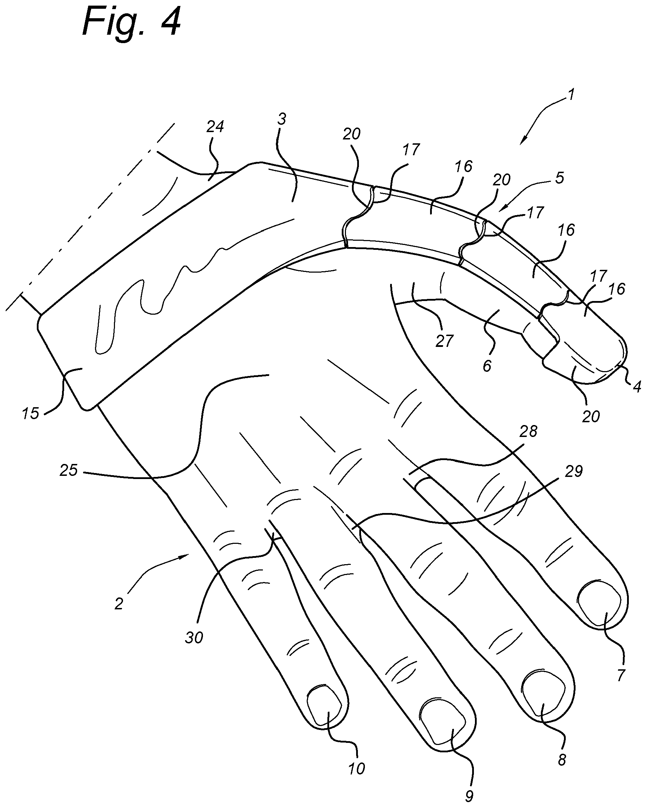

Figure 4 shows a view to the dorsal side 12 of the hand 2 of a second embodiment of the finger protection device 1. In this embodiment the hand brace element 3 covers and supports the carpal region 24 of the hand 2. The finger brace structure 19 extends from the hand brace element 3 and includes the finger top cap 4 and the connecting element 5.

The connecting element 5 is formed from connecting element parts 16 that are mutually connected through moveable connections 17, such as a hinge. The hinges 17 allow the connecting element parts 16 to be moved with respect to each other and the hand brace element 3 when the thumb 6 is moved towards the hand palm 11. The connecting element parts 16 each cover the dorsal side of a phalanx of the thumb 6. Additionally, the connecting element part 16 covers part of the medial and lateral sides of the phalanx. The connecting part 16 that covers the distal phalanx of the thumb is integrated with the finger top cap 4. The finger top cap 4 accommodates part of the distal phalanx, such that the thumb 6 can move the finger brace structure 19 when an object is held or upon forming a fist.

A blocking element 20 provided at each hinge 17 prevents the pivoting of the hinges 17 when a force moves the thumb 6 towards the dorsum 12 of the hand 2. In addition, the blocking element 20 provides rigidity to the hinges 17 when a force parallel to the connecting element in flexion of the thumb 6 is exerted on the finger top cap 4.

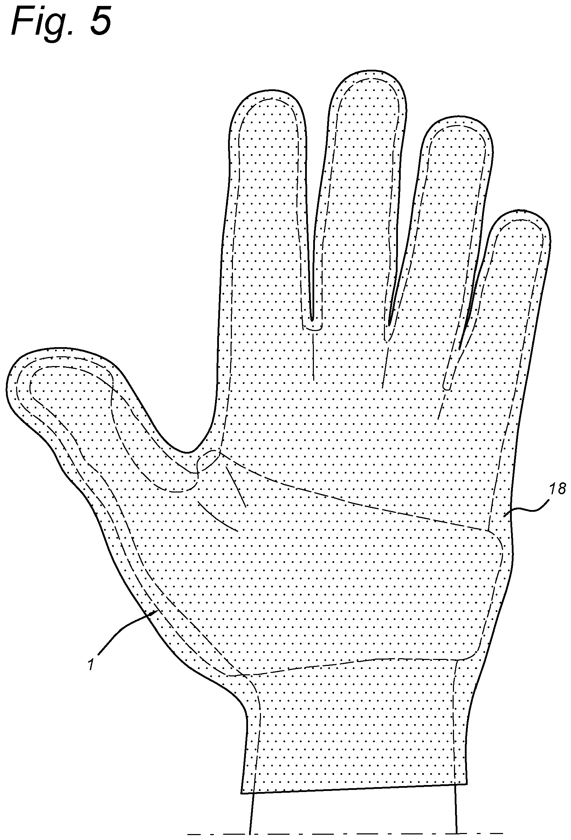

Figure 5 shows a glove 18 with an inserted finger protection device 1. The finger protection device 1 can be integrated into the glove 18 during manufacturing, but can also be inserted after both the glove 18 and the device 1 have been manufactured separately. Alternatively, the finger protection device can be put over the glove or the glove can be manufactured in a ready-made device. The finger top cap 4 of the finger protection device 1 is accommodated in the thumb part 35 of the glove 18. The hand brace element 3 is accommodated in the hand part 36 of the glove 18. When wearing the glove 18, the carpal region 24 and the metacarpal region 25 of the hand 2 of the user is supported by the hand brace element 3 and the thumb 6 is protected and supported by the finger brace structure 19.

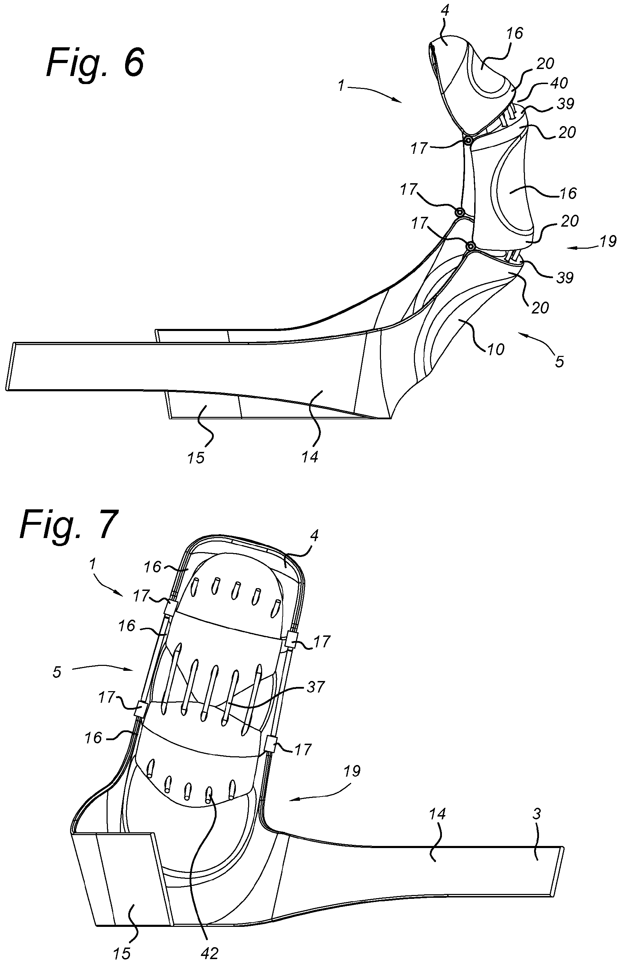

Figures 6 and 7 show a third embodiment of the finger protection device 1. The finger brace structure 19 comprises the connecting element 5, which in turn comprises connecting element part 16. The connecting element part 16 at the distal phalanx is connected to the finger top cap 4. The movable connection is schematically shown by reference number 17 between the connecting element parts 16 and is provided at the respective ends of the connecting element parts, preferably by piping (not shown) provided along the respective edges of the connecting element parts and extending from the connecting element part covering the distal phalanx in use to the hand brace element. The movable connection between the connecting element parts 16 is furthermore established by five wires 37, see Figure 7. Any number of wires 37 can be used, but three or more is preferred, such as five or seven elongated connection members. The larger the number of wires 37 used, the higher the resistance to torsion of the finger brace structure 19 will be. The wires 37 in Figs. 6-8 are connected at a first end to the first connecting element part 16 of the connecting element 5 attached to the hand brace element 3 and with a second end to the connecting element part 16 covering the distal phalanx.

Figures 7 an 8b show that the wires are slidably received in through holes 41 forming receiving openings 38 in the respective connecting element parts. Upon movement of the thumb, the connecting element parts can move with respect to each other by the wires sliding in the through holes 41. The length of the wires is such that upon normal movement of the thumb, at least the ends 42 of the wires will remain in the through holes to retain the movable connection 17. The end 42 of the wires can be made such that the movement of the elongated connection members within the through holes 41 will be blocked with a blocking member (not shown).

The connecting element parts 16 are provided with blocking elements 20 that are formed as relatively thicker portions at the ends of the connecting element parts 16. The relatively thicker portions are provided with abutment surfaces 39 where the connecting element parts 16 abut each other upon movement of the thumb away from the palm of the hand, such that overstretching is prevented.

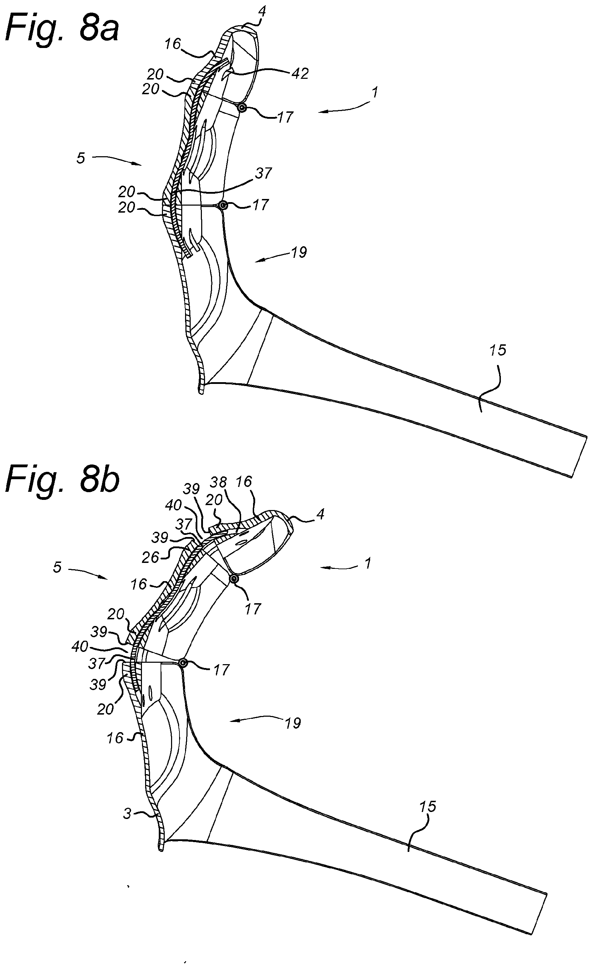

Figure 8 shows cross-sectional views of the third embodiment of the finger protection device 1 in two distinct positions of the connecting element parts. Figure 8a shows the finger brace structure 19 in its closed position, i.e. the position of the connecting element parts 16 upon movement of the thumb away from the palm of the hand. As shown, the blocking elements 20 abut at the abutment surfaces 39 to block the finger brace structure 19 from further movement away from the palm and prevent overstretching of the thumb. Figure 8b shows the finger brace structure 19 in its open position, i.e. the position of the connecting element parts 16 upon movement of the thumb towards the palm of the hand. In this position, slits 40 between the connecting element parts 16 are generated. Preferably, these slits 40 are closed by a flexible sheet covering at least the slits (not shown). Closure of the slits 40 prevent snow and ice to enter the finger brace structure 19 and block the movement of the finger brace structure 19. The flexible sheet can extend from the connecting element part 16 attached to the hand brace element 3 to the connecting element part 16 covering the distal phalanx.

**LIST OF PARTS**

| | | | |
|---|---|---|---|
| 1. | Finger protection device | 38. | Receiving opening |
| 2. | Hand | 39. | Abutment surface |
| 3. | Hand brace element | 40. | Slit |
| 4. | Thumb top cap | 41. | Through hole |
| 5. | Connecting element | 42. | Wire end |
| 6. | Thumb | | |
| 7. | Index finger | | |
| 8. | Middle finger | | |
| 9. | Ring finger | | |
| 10. | Small finger or pinky | | |
| 11. | Palm of hand | | |
| 12. | Dorsal side of hand | | |
| 13. | Wrist | | |
| 14. | Palmar side of hand brace element | | |
| 15. | Dorsal side of hand brace element | | |
| 16. | Connecting element part | | |
| 17. | Moveable connection | | |
| 18. | Glove | | |
| 19. | Finger brace structure | | |
| 20. | Blocking element | | |
| 21. | Entry opening | | |
| 22. | Thumb exit opening | | |
| 23. | Grouped finger opening | | |
| 24. | Carpal region of hand | | |
| 25. | Metacarpal region of hand | | |
| 26-30. | Web space between two fingers | | |
| 31. | Adjustment element | | |
| 32. | Base of U-profile | | |
| 33+34. | Flange of U-profile | | |
| 35. | Thumb part of glove | | |
| 36. | Hand part of glove | | |
| 37. | Elongated connection member | | |

## Claims

1. Finger protection device (1), comprising
- a hand brace element (3) for accommodating at least a carpal region of a hand (2), and
- at least one finger brace structure (19) comprising a finger top cap (4) to accommodate a finger top, and a flexible connecting element (5) extending between the hand brace element and the finger top cap, the connecting element being sufficiently rigid to transmit an axial first force applied to the finger top cap to the hand brace element, **characterised in that**, the hand brace element has an entry opening (21) for inserting the hand into the hand brace element and wherein a web space part (26) is provided to cover in use at least part of a web space (27) between at least the thumb (6) and the index finger (7), the web space part forming at least two exit openings (22, 23) for extension of the fingers of the hand in use,
and the finger top cap extends from the top of the distal phalanx to the web space part to provide a closure of the connecting element at the palmar side of the finger.

2. Device (1) according to claim 1, wherein the finger brace structure (19) is sufficiently rigid to transmit a transverse second force directed to the palmar side of the connecting element (5), to the hand brace element (3), and/or wherein the finger brace structure is relatively rigid in an axial direction of the connecting element to deflect the axial first force to the hand brace element, and relatively flexible in a transverse direction directed to a dorsal side of the connecting element to move the finger towards the palm of the hand.

3. Device (1) according to preceding claim 1 or 2, comprising a pivotable connection (17) between the connecting element (5) and the hand brace element (3) for allowing pivoting of the finger (6-10) in a direction towards a palm (11) of the hand.

4. Device (1) according to any of the preceding claims, wherein the connecting element (5) comprises a U-shaped profile covering the dorsal side of the phalanges and at least part of a lateral and at least part of a medial side of the phalanges of the finger.

5. Device (1) according to any of the preceding claims, wherein the at least two exit openings (22, 23) comprise an exit opening (22) for the thumb (6) and an exit opening (23) for grouped fingers (7-10).

6. Device (1) according to any of the preceding claims, wherein the connecting element (5) comprises a connecting element part (16) for each phalanx in a finger, wherein the connecting element part in use covering a distal phalanx of a finger is connected to the finger top cap, and wherein the connecting element parts are connected to each other by a pivotable connection, such that the connecting element parts are pivotable with respect to each other when in use a transverse third force directed to a dorsal side of the finger brace structure is exerted on the finger brace structure..

7. Device (1) according to any of the preceding claims, wherein the pivotable connection between the connecting element parts is established by at least one flexible elongated connection member connected with a first and a second end to respective connection element parts, preferably wherein the connection member extends between the connecting element and the hand brace element.

8. Device (1) according to any of the preceding claims, wherein the pivotable connection (17) between the connecting element parts (16) is established by a connecting strip provided at the respective edges of the connecting element parts, preferably wherein the pivotable connection extends between the connecting element and the hand brace element (3), and wherein the connecting strip extends along the edges of the connecting element parts from the hand brace element to the finger top cap (4).

9. Device (1) according to any of the preceding claims, wherein at least one pivotable connection comprises a blocking element (20) blocking pivoting of the pivotable connection beyond a flexed position of the hand in response to a force on the finger towards the dorsal side of the hand, preferably wherein the blocking element is formed at the opposite longitudinal ends of a respective connecting element part by a relatively thicker portion extending along the ends of the connecting element part.

10. Device (1) according to any of the preceding claims, wherein the finger brace structure is provided at the thumb opening for protection of the thumb only.

11. Device (1) according to any of the preceding claims, wherein the hand brace element is sufficiently rigid to receive the axial first force and the transverse third force from the finger brace structure.

12. Device (1) according to any of the preceding claims, wherein the hand brace element is sufficiently rigid to support at least the carpal region of the hand.

13. Device (1) according to any one of claims 1 - 12, which is formed as a thumb protection device, wherein the finger brace structure (19) forms a thumb brace structure and the finger top cap (4) forms a thumb top cap for accommodating a top of a thumb (6).

14. Glove (18) comprising a finger protection device (1) according to any of claims 1-13.

15. Glove (18) according to claim 14, wherein the device (1) and the glove form an integral unit.

## Patentansprüche

1. Fingerschutzvorrichtung (1), umfassend:
- ein Handverstärkungselement (3) zum Aufnehmen wenigstens eines Handwurzelknochenbereichs einer Hand (2), und
- wenigstens eine Fingerverstärkungsstruktur (19) umfassend eine Fingerspitzenkappe (4), um eine Fingerspitze aufzunehmen, und ein flexibles Verbindungselement (5), das sich zwischen dem Handverstärkungselement und der Fingerspitzenkappe erstreckt, wobei das Verbindungselement ausreichend steif ist, um eine axiale erste Kraft, die auf die Fingerspitzenkappe beaufschlagt wird, zu dem Handverstärkungselement zu übertragen,
**dadurch gekennzeichnet, dass**
das Handverstärkungselement eine Eintrittsöffnung (21) zum Insertieren der Hand in das Handverstärkungselement aufweist, und wobei ein Bahnraumteil (26) bereitgestellt ist, um bei Verwendung wenigstens einen Teil eines Bahnraums (27) zwischen wenigstens dem Daumen (6) und dem Zeigefinger (7) abzudecken, wobei das Bahnraumteil wenigstens zwei Austrittsöffnungen (22, 23) zur Streckung der Finger der Hand bei Verwendung bildet,
und wobei die Fingerspitzenkappe sich von der Oberseite des distalen Fingerglieds zu dem Bahnraumteil erstreckt, um einen Verschluss des Verbindungselements an der Hohlhandseite des Fingers bereitzustellen.

2. Vorrichtung (1) nach Anspruch 1, wobei die Fingerverstärkungsstruktur (19) ausreichend steif ist, um eine transversale zweite Kraft, die auf die Hohlhandseite des Verbindungselements (5) gerichtet ist, zu dem Handverstärkungselement (3) zu übertragen, und/oder wobei die Fingerverstärkungsstruktur verhältnismäßig steif in einer axialen Richtung des Verbindungselements ist, um die axiale erste Kraft zu dem Handverstärkungselement abzulenken, und verhältnismäßig flexibel in einer transversalen Richtung, die auf eine dorsale Seite des Verbindungselements gerichtet ist, ist, um die Finger in Richtung auf die Handfläche der Hand zu bewegen.

3. Vorrichtung (1) nach vorangehendem Anspruch 1 oder 2, umfassend eine drehbare Verbindung (17) zwischen dem Verbindungselement (5) und dem Handverstärkungselement (3), um ein Drehen des Fingers (6-10) in einer Richtung auf eine Handfläche (11) der Hand zu erlauben.

4. Vorrichtung (1) nach einem der vorangehenden Ansprüche, wobei das Verbindungselement (5) ein U-förmiges Profil umfasst, das die dorsale Seite der Fingerglieder und wenigstens einen Teil einer seitlichen und wenigstens einen Teil einer medialen Seite der Fingerglieder des Fingers abdeckt.

5. Vorrichtung (1) nach einem der vorangehenden Ansprüche, wobei die wenigstens zwei Austrittsöffnungen (22, 23) eine Austrittsöffnung (22) für den Daumen (6) und eine Austrittsöffnung (23) für gruppierte Finger (7-10) umfasst.

6. Vorrichtung (1) nach einem der vorangehenden Ansprüche, wobei das Verbindungselement (5) ein Verbindungselementteil (16) für jedes Fingerglied in einem Finger umfasst, wobei das Verbindungselementteil, das bei Verwendung ein distales Fingerglied eines Fingers abdeckt, mit der Fingerspitzenkappe verbunden ist, und wobei die Verbindungselementteile miteinander durch eine drehbare Verbindung verbunden sind, so dass die Verbindungselementteile in Bezug zueinander drehbar sind, wenn bei Verwendung eine transversale dritte Kraft, gerichtet auf eine dorsale Seite der Fingerverstärkungsstruktur, auf die Fingerverstärkungsstruktur ausgeübt wird.

7. Vorrichtung (1) nach einem der vorangehenden Ansprüche, wobei die drehbare Verbindung zwischen den Verbindungselementteilen durch wenigstens ein flexibles längliches Verbindungsglied begründet ist, das mit einem ersten und einem zweiten Ende an entsprechende Verbindungselementteile angebunden ist, wobei bevorzugt das Verbindungsglied sich zwischen dem Verbindungselement und dem Handverstärkungselement erstreckt.

8. Vorrichtung (1) nach einem der vorangehenden Ansprüche, wobei die drehbare Verbindung (17) zwischen den Verbindungselementteilen (16) durch einen Verbindungsstreifen begründet ist, bereitgestellt an den entsprechenden Rändern der Verbindungselementteile, wobei bevorzugt die drehbare Verbindung sich zwischen dem Verbindungselement und dem Handverstärkungselement (3) erstreckt, und wobei sich der Verbindungsstreifen entlang der Ränder der Verbindungselementteile von dem Handverstärkungselement zu der Fingerspitzenkappe (4) erstreckt.

9. Vorrichtung (1) nach einem der vorangehenden Ansprüche, wobei wenigstens eine drehbare Verbindung ein Blockierelement (20) umfasst, das ein Drehen der drehbaren Verbindung über eine geknickte Position der Hand in Antwort auf eine Kraft auf den Finger in Richtung auf die dorsale Seite der Hand hinaus blockiert, wobei bevorzugt das Blockierelement an den gegenüberliegenden Längsenden eines entsprechenden Verbindungselementteils durch einen verhältnismäßig dickeren Bereich, der sich entlang der Enden des Verbindungselementteils erstreckt, gebildet ist.

10. Vorrichtung (1) nach einem der vorangehenden Ansprüche, wobei die Fingerverstärkungsstruktur an der Daumenöffnung zum Schutz lediglich des Daumens bereitgestellt ist.

11. Vorrichtung (1) nach einem der vorangehenden Ansprüche, wobei das Handverstärkungselement ausreichend steif ist, um die axiale erste Kraft und die transversale dritte Kraft von der Fingerverstärkungsstruktur aufzunehmen.

12. Vorrichtung (1) nach einem der vorangehenden Ansprüche, wobei das Handverstärkungselement ausreichend steif ist, um wenigstens den Handwurzelknochenbereich der Hand zu stützen.

13. Vorrichtung (1) nach einem der Ansprüche 1 bis 12, welche als eine Daumenschutzvorrichtung gebildet ist, wobei die Fingerverstärkungsstruktur (19) eine Daumenverstärkungsstruktur bildet und die Fingerspitzenkappe (4) eine Daumenspitzenkappe zum Aufnehmen einer Spitze eines Daumens (6) bildet.

14. Handschuh (18) umfassend eine Fingerschutzvorrichtung (1) nach einem der Ansprüche 1 bis 13.

15. Handschuh (18) nach Anspruch 14, wobei die Vorrichtung (1) und der Handschuh eine integrale Einheit bilden.

## Revendications

1. Dispositif de protection de doigts (1) comprenant
- un élément formant attelle manuelle (3) destiné à recevoir au moins la région carpienne d'une main (2) et
- au moins une structure formant attelle digitale (19) comprenant un capuchon de bout de doigt (4) destiné à recevoir un bout de doigt, et un élément de liaison flexible (5) s'étendant entre l'élément formant attelle manuelle et le capuchon de bout de doigt, l'élément de liaison étant suffisamment rigide pour transmettre une première force axiale appliquée sur le capuchon de bout de doigt à l'élément formant attelle manuelle,
**caractérisé en ce que**
l'élément formant attelle manuelle possède une ouverture d'entrée (21) destinée à insérer la main dans l'élément formant attelle manuelle et une partie interdigitale (26) est prévue pour recouvrir en utilisation au moins une partie d'un espace interdigital (27) situé entre au moins le pouce (6) et l'index (7), la partie interdigitale formant au moins deux ouvertures de sortie (22, 23) destinées à l'extension des doigts de la main en cours d'utilisation,
et le capuchon de bout de doigt s'étend du bout de la phalange distale à la partie interdigitale pour assurer la fermeture de l'élément de liaison du côté palmaire du doigt.

2. Dispositif (1) selon la revendication 1, dans lequel la structure formant attelle digitale (19) est suffisamment rigide pour transmettre une deuxième force transversale dirigée vers le côté palmaire de l'élément de liaison (5), vers l'élément formant attelle manuelle (3) et/ou la structure formant attelle digitale est relativement rigide dans une direction axiale de l'élément de liaison pour dévier la première force axiale vers l'élément formant attelle manuelle et relativement flexible dans une direction transversale dirigée vers un côté dorsal de l'élément de liaison pour déplacer le doigt vers la paume de la main.

3. Dispositif (1) selon la revendication précédente 1 ou 2, comprenant une liaison pivotante (17) entre l'élément de liaison (5) et l'élément formant attelle manuelle (3) pour permettre le pivotement du doigt (6-10) en direction de la paume (11) de la main.

4. Dispositif (1) selon l'une quelconque des revendications précédentes, dans lequel l'élément de liaison (5) comprend un profil en forme de U, recouvrant le côté dorsal des phalanges et au moins une partie d'un côté latéral et au moins une partie d'un côté médian des phalanges du doigt.

5. Dispositif (1) selon l'une quelconque des revendications précédentes, dans lequel les au moins deux ouvertures de sortie (22, 23) comprennent une ouverture de sortie (22) destinée au pouce (6) et une ouverture de sortie (23) destinée aux doigts regroupés (7-10).

6. Dispositif (1) selon l'une quelconque des revendications précédentes, dans lequel l'élément de liaison (5) comprend une partie d'élément de liaison (16) destinée à chaque phalange d'un doigt, dans lequel la partie d'élément de liaison recouvrant en utilisation une phalange distale d'un doigt est reliée au capuchon de bout de doigt, et dans lequel les parties d'élément de liaison sont reliées entre elles par une liaison pivotante, de sorte que les parties d'élément de liaison peuvent pivoter les unes par rapport aux autres lorsque, en utilisation, une troisième force transversale dirigée vers un côté dorsal de la structure formant attelle digitale est exercée sur la structure formant attelle digitale.

7. Dispositif (1) selon l'une quelconque des revendications précédentes, dans lequel la liaison pivotante entre les parties d'élément de liaison est réalisée par au moins un élément de liaison allongé flexible relié par des première et seconde extrémités à des parties d'élément de liaison respectives, de préférence l'élément de liaison s'étendant entre l'élément de liaison et l'élément formant attelle manuelle.

8. Dispositif (1) selon l'une quelconque des revendications précédentes, dans lequel la liaison pivotante (17) entre les parties d'élément de liaison (16) est réalisée par une bande de liaison prévue au niveau des bords respectifs des parties d'élément de liaison, de préférence la liaison pivotant s'étendant entre l'élément de liaison et l'élément formant attelle manuelle (3) et la bande de liaison s'étendant le long des bords des parties d'élément de liaison depuis l'élément formant attelle manuelle jusqu'au capuchon de bout de doigt (4).

9. Dispositif (1) selon l'une quelconque des revendications précédentes, dans lequel au moins une liaison pivotante comprend un élément de blocage (20) bloquant le pivotement de la liaison pivotante au-delà d'une position fléchie de la main en réponse à une force exercée sur le doigt vers le côté dorsal de la main, de préférence l'élément de blocage étant formé, au niveau d'extrémités longitudinales opposées d'une partie d'élément de liaison respective, par une portion relativement plus épaisse s'étendant le long des extrémités de la partie d'élément de liaison.

10. Dispositif (1) selon l'une quelconque des revendications précédentes, dans lequel la structure d'attelle de doigt est prévue au niveau de l'ouverture de pouce pour protéger uniquement le pouce.

11. Dispositif (1) selon l'une quelconque des revendications précédentes, dans lequel l'élément formant attelle manuelle est suffisamment rigide pour recevoir la première force axiale et la troisième force transversale de la structure formant attelle digitale.

12. Dispositif (1) selon l'une quelconque des revendications précédentes, dans lequel l'élément formant attelle manuelle est suffisamment rigide pour supporter au moins la région carpienne de la main.

13. Dispositif (1) selon l'une quelconque des revendications 1 à 12, qui est réalisé sous la forme d'un dispositif de protection de pouce, la structure formant attelle digitale (19) formant une structure formant attelle de pouce et le capuchon de bout de doigt (4) formant un capuchon de bout de pouce destiné à recevoir le bout d'un pouce (6).

14. Gant (18) comprenant un dispositif de protection de doigts (1) selon l'une quelconque des revendications 1 à 13.

15. Gant (18) selon la revendication 14, dans lequel le dispositif (1) et le gant forment une unité d'une seule pièce.
